# EUROPEAN PATENT APPLICATION

(11) **EP 1 698 376 A1**
(43) Date of publication of application: **06.09.2006**
(21) Application number: 06250913.8
(22) Date of filing: 21.02.2006
(51) Int. Cl.: A61Q 3/02, A61K 8/81

(54) **Aqueous nail varnish with improved film properties**

(30) Priority: 03.03.2005 EP 05290484
(71) Applicant: Rohm and Haas Company, Philadelphia, PA 19106-2399 (US)
(72) Inventor: Duccini, Yves, 06250 Mougins (FR); Sanfilippo, Angelo, Les Villas De Mandelieu No. 67, 06210 Mandelieu (FR); Ugazio, Stepahen P.J., 7100 La Louviere (BE)
(74) Representative: Kent, Venetia Katherine

(57) **Abstract**

A method of coating human nails with a polymer film. The method comprises steps of: (a) applying to the human nails a composition comprising:
(i) a first polymer binder having M_{w} greater than 20,000 and a T_{g} less than 70°C;
(ii) a second polymer binder having M_{w} less than 15,000 and a T_{g} greater than 90°C; and (iii) water; wherein the composition contains less than 10% of organic solvents; and (b) allowing the composition to dry until the polymer film has formed.

## Description

The present invention relates to a method for coating human nails with a polymer film. The method employs an aqueous composition which has fast drying properties.

U.S. Pat. No. 5,965,111 discloses a fast drying water-based nail varnish formulation. However, this formulation contains at least 20% by weight of a volatile organic solvent. Nail varnish formulations which contain water tend to have lower gloss and film hardness than traditional solvent-borne formulations. Another problem with aqueous formulations is that the dried film is not easily removed with water.

The problem addressed by this invention is the need for an aqueous nail varnish that contains lower amounts of volatile organic solvents, especially those known to be detrimental to the environment and/or human health, commonly known as "VOC" solvents, and which is more easily removed with water.

### STATEMENT OF THE INVENTION

The present invention is directed to a method of coating human nails with a polymer film; said method comprising steps of (a) applying to the human nails a composition comprising: (i) a first polymer having M_{w} greater than 20,000 and a Tg less than 70°C; (ii) a second polymer having M_{w} less than 15,000 and a Tg greater than 90°C; and (iii) water; wherein the composition contains less than 10% of organic solvents; and (b) allowing the composition to dry until the polymer film has formed.

The present invention is further directed to an aqueous composition suitable for use as a nail polish comprising: (a) a first polymer having M_{w} greater than 20,000 and a T_{g} less than 70°C; (b) a second polymer having M_{w} less than 15,000 and a T_{g} greater than 90°C; and (c) water; wherein the composition contains less than 10% of organic solvents and less than 10% inorganic material; and wherein the composition comprises 40-96% of the first polymer and 4-60% of the second polymer, based on dry weight of polymeric material in the composition.

### DETAILED DESCRIPTION OF THE INVENTION

The term "human nail" refers to a fingernail or toenail of a human being. All percentages and ppm values are on the basis of total weight of the composition, unless otherwise indicated. The term "acrylic polymers" refers to polymers comprising at least 50% monomer units derived from among acrylic acid (AA), methacrylic acid (MAA) and their esters. Esters of AA and MAA include, but are not limited to, methyl methacrylate (MMA), ethyl methacrylate (EMA), butyl methacrylate (BMA), hydroxyethyl methacrylate (HEMA), acetylacetoxyethyl methacrylate (AAEM), methyl acrylate (MA), ethyl acrylate (EA), butyl acrylate (BA), and hydroxyethyl acrylate (HEA), as well as other esters of AA or MAA, e.g., alkyl, hydroxyalkyl and aminoalkyl esters. Acrylic polymers also may contain monomer units derived from other ethylenically unsaturated monomers, e.g., styrene or substituted styrenes; other α,B-unsaturated carboxylic acids, esters and amides; vinyl esters or halides; etc. Preferably, an acrylic polymer contains less than 30% of these other monomer units, more preferably less than 10%, and most preferably the acrylic polymers are substantially free of monomer units other than those of AA, MA and their esters. An "acrylic-styrene copolymer" is a polymer at least 50% of whose monomer units are derived from among AA, MAA, esters of AA and MAA, and styrene monomers. Styrene monomers include styrene (Sty) and substituted styrenes, e.g., α-methylstyrene (AMS). Preferably, acrylic-styrene copolymers contain less than 20% of other monomer units, more preferably less than 10%, and most preferably less than 5%. The term "inorganic" refers to materials that do not contain carbon, with the exceptions that metal salts containing carbonate are considered to be inorganic, and water and ammonia are not considered to be inorganic.

Preferably, the composition comprises 40-96% of the first polymer and 4-60% of the second polymer, based on dry weight of polymeric material in the composition. Either the first or the second polymer, or both, can be a mixture of different polymer compositions, providing that they meet the M_{w} and T_{g} requirements. The first polymer, which is a lower-Tg polymer, is also referred to herein as the "soft-type" polymer, and the second polymer as the "hard-type" polymer. In one preferred embodiment of the invention, the composition comprises 40-90% of the first polymer and 10-60% of the second polymer, based on dry weight of polymeric material in the composition. In another preferred embodiment, the composition comprises 40-80% of the first polymer and 20-60% of the second polymer, based on dry weight of polymeric material in the composition.

In the composition of this invention, the first polymer has M_{w} greater than 20,000 and a Tg less than 70°C, and the second polymer has M_{w} less than 15,000 and a Tg greater than 90°C. More preferably, the first polymer has M_{w} greater than 25,000 and a T_{g} less than 60°C. More preferably, the second polymer has M_{w} less than 10,000 and a Tg greater than 100°C. Most preferably, the first polymer has M_{w} greater than 35,000 and a Tg less than 50°C. Most preferably, the second polymer has M_{w} less than 7,000 and a Tg greater than 110°C. In one embodiment of the invention, the first polymer has M_{w} greater than 100,000 and a Tg less than 70°C, and more preferably has M_{w} greater than 200,000 and a Tg less than 60°C. T_{g} for the first polymer is measured using Differential Scanning Calorimetry with standard techniques for calculation of T_{g} values. For the second polymer, T_{g} is calculated as the simple linear average of the T_{g} values for homopolymers of each monomer whose residue is contained in the second polymer, with monomer concentrations as weighting factors ("linear Tg"). Preferably, the polymers are acrylic polymers or acrylic-styrene copolymers.

In one embodiment of the invention, the aqueous compositions contain an acid-containing latex; that is, the polymer in the latex has pendant carboxylic acid groups. The addition of acid functional groups is believed, without reliance thereon, to enhance the stability of the composition. Acid-containing latexes are well known to those skilled in the art, and their preparation will not be further discussed herein.

In one embodiment of the invention, the composition contains at least one polyurethane binder. The polyurethane binder comprises, for example, a polyether polyurethane, a polyester polyurethane, or a combination thereof. The polyurethane binder may be aliphatic, aromatic, or a combination thereof. Preferably, the polyurethane binder is present in an amount no more than 50%, based on weight of the polyurethane solids as a percentage of the total polymer weight in the composition, more preferably no more than 30%, and most preferably no more than 20%.

Preferably, the polymer content of the composition, measured as dry polymer, is from 20-60%, more preferably from 30-50%.

Surfactants are commonly used in emulsion or dispersion polymerization to provide stability, as well as to control particle size. Surfactants can also provide dispersibility for water-reducible resins. Conventional surfactants include anionic or nonionic emulsifiers or combinations thereof. Typical anionic emulsifiers include but are not limited to: alkali or ammonium alkyl sulfates, alkyl sulfonates, salts of fatty acids, esters of sulfosuccinic acid salts, alkyl diphenylether disulfonates, and salts or free acids of complex organic phosphate esters. Typical nonionic emulsifiers include but are not limited to: polyethers, e.g. ethylene oxide and propylene oxide condensates which include straight and branched chain alkyl and alkylaryl polyethylene glycol and polypropylene glycol ethers and thioethers, alkyl phenoxypoly(ethyleneoxy) ethanols having alkyl groups containing from about 7 to about 18 carbon atoms and having from about 4 to about 100 ethyleneoxy units, and polyoxyalkylene derivatives of hexitol, including sorbitans, sorbides, mannitans, and mannides. Surfactants may be employed in the compositions of the present invention at levels of 0.05 - 1 wt% or greater, based on the total weight of the final composition.

The aqueous compositions of the present invention may optionally contain additional components including but not limited to: thickeners; rheology modifiers; dyes; sequestering agents; biocides; dispersants; colorants such as the typical organic dyes and inorganic pigments used in the cosmetics and paint industries; plasticizers; adhesion promoters; coalescents; wetting agents; waxes; surfactants; slip additives; crosslinking agents; defoamers; preservatives; perfumes (at 0.05% to 1%); freeze/thaw protectors; and alkali or water soluble polymers, including other binders that can increase film hardness, adhesion and water resistance, such as polyurethanes or classical- or core-shell-type latexes. In one embodiment of the invention, the aqueous composition contains 1-10% of a wax, more preferably from 1.5-8%, and most preferably from 2-6%. Polyolefin waxes are preferred.

Preferably, the aqueous compositions contain less than 15% of inorganic material, more preferably less than 10%. In one embodiment of the invention, they contain less than 7% inorganic material. In another embodiment of the invention, the aqueous composition is substantially free of inorganic material; preferably in this embodiment, the nail varnish is colorless and clear, or at most slightly hazy. Inorganic materials that may be used in the composition include, for example, inorganic pigments and colored inorganic particles.

The aqueous compositions used in the present invention contain less than 10% of organic solvents. Preferably the compositions contain less than 8% of organic solvents, and most preferably less than 7%. Preferably the aqueous compositions contain less than 4% of VOC solvents, more preferably less than 2%, and most preferably the compositions are substantially free of VOC solvents. VOC solvents are those organic solvents that have non-negligible atmospheric photochemical reactivity. The term "VOC solvent" is defined in readily accessible environmental regulations in most jurisdictions.

As this nail varnish is aqueous, it can be prepared easily in situ (e.g., in shops) by adding to a partially formulated aqueous polymer binder any colorant or pigments or perfumes preferred by the customer. Hence a customer could establish the color and fragrance of the nail varnish, and the vendor could prepare the desired nail varnish accordingly from a base formulation not containing these ingredients.

### EXAMPLES

### Example 1

| Ingredients / sample ref. | A1 | A2 | A3 | A4 | A5 |
|---|---|---|---|---|---|
| Binder A (40%) | 186.8 | 186.8 | 186.8 | 186.8 | 186.8 |
| Solvent | 12.6 | 12.6 | 12.6 | 12.6 | 12.6 |
| Binder B (30%) | | 22.0 | 44.0 | 0.0 | 0.0 |
| Binder C (45%) | | | | 14.7 | 29.4 |
| Thickener QR2020 | 0.6 | 3.6 | 3.6 | 3.6 | 3.6 |
| **Total** | **200.0** | **225.0** | **247.0** | **217.7** | **232.4** |
| | | | | | |
| Polymer "soft-type A" dry | 74.7 | 74.7 | 74.7 | 74.7 | 74.7 |
| Hardener "hard-type 1" dry | | 6.6 | 13.2 | | |
| Hardener "hard-type 2" dry | 0.0 | | | 6.6 | 13.2 |
| **Total dry** | **74.7** | **81.3** | **87.9** | **81.3** | **88.0** |
| | | | | | |
| Polymer "soft-type A" dry % | 100.0 | 91.9 | 85.0 | 91.9 | 85.0 |
| Hardener "hard-type 1" dry % | | 8.1 | 15.0 | | |
| Hardener "hard-type 2" dry % | | | | 8.1 | 15.0 |
| **Total dry %** | **100.0** | **100.0** | **100.0** | **100.0** | **100.0** |

Notes
1. Binder A was a 40% solids aqueous binder containing a crosslinked 53.7:21.3:11.4:8.3:5.2, MMA/EHA/BA/AAEM/MAA polymer, with a Tg of 44°C, and M_{w} approximately 250,000.
2. Binder B was a 29% solids aqueous binder containing a 36:35:28, AMS/Sty/AA polymer with a linear Tg of 116°C, acid number 205, and M_{w} 6500.
3. Binder C was a 45% solids aqueous binder containing a 31:35:33, AMS/Sty/AA polymer with a linear Tg of 114.55°C, acid number 235, and M_{w} 1200.
4. The solvent was DOWANOL DPnB {1-(2-butoxy-2-methylethoxy)-2-propanol; available from Dow Chemical Co.}
6. The thickener was QR2020 (available from Rohm and Haas Co., Philadelphia, PA), a polyurethane thickener.

### FILM APPLICATION = 90 µ WET

| HARDNESS Konig 6°- 3° | | | | | |
|---|---|---|---|---|---|
| Drying time = 1H | 25 | 41 | 40 | 28 | 30 |
| Drying time = 24 H | 65 | 95 | 95 | 72 | 73 |
| Drying time = 48 H | 84 | 110 | 108 | 84 | 86 |
| | | | | | |

| Water resistance (time min.) | | | | | |
|---|---|---|---|---|---|
| Drying time = 1 H | 6 | 5 | 3 | 2 | 1 |
| Drying time = 24 H | 20 | 20 | 20 | 10 | 4 |
| Drying time = 48 H | 20 | 20 | 20 | 11 | 4 |

### FILM APPLICATION = 30 µ WET

| HARDNESS König 6°- 3° | | | | | |
|---|---|---|---|---|---|
| Drying time = 1 H | 79 | 120 | 120 | 96 | 95 |
| Drying time = 24 H | * | | | | |
| Drying time = 48 H | * | | | | |

| | | | | | |
|---|---|---|---|---|---|
| * not measured | | | | | |

| Water resistance (time min.) | | | | | | |
|---|---|---|---|---|---|---|
| Drying time = 1 H | 4 | | 3 | 2 | 2 | 1 |
| Drying time = 24 H | 5 | | 5 | 3 | 2 | 1 |
| Drying time = 48 H | 5 | | 5 | 3 | 2 | 1 |

### Gloss Measurement

To the previous formulation 1.5% of a red colorant slurry from CPS Color was added so that gloss can be evaluated.

| **Angle** | **20°** | | **60°** | | **85°** | |
|---|---|---|---|---|---|---|
| **Substrate** | **white** | **black** | **white** | **black** | **white** | **black** |
| sample | **gloss** | **gloss** | **gloss** | **gloss** | **gloss** | **gloss** |
| A1 | **76.6** | 76.2 | **86.6** | 86.5 | **98.5** | 98.9 |
| A2 | **76.3** | 76.2 | **86.9** | 86.9 | **98.5** | 98.4 |
| A3 | **78.6** | 78.6 | **88** | 87.7 | **98.8** | 98.4 |
| A4 | **79.6** | 78.5 | **85** | 84.9 | **97.9** | 98.4 |
| A5 | **81.8** | 81.7 | **85.3** | 85 | **96.6** | 97.6 |

These data demonstrate that, through addition of Binder B or Binder C, the film properties of hardness and gloss could be improved, and the water resistance decreased so that the coating is easier to remove.

König Hardness is measured by ASTM test method D4366.

Gloss is measured by ASTM test method D523.

Water Resistance is measured as follows. Using a specific application, draw down a 30 micron or 90 micron film of wet nail varnish on five glass plates. Leave to dry at room temperature for 1 hour, or 24 hours or 48 hours Test at 20°C:
Immerse the glass plates in water at 20°C. Whilst immersed in the water, gently scratch the surface of the varnish with a pencil after 1 minute. If the varnish peels off the glass plate, record the water resistance after 1 hour dry time at 20°C (WR/1 hr/20°C) as being less than 1 minute. Otherwise continue the test until the varnish peels off the surface (generally less than 20 minutes). Repeat test for glass plates with 24 hours and 48 hrs dry time.

### Example 2

| Ingredients / sample ref. | A1 | A2 | A3 | A4 | A5 |
|---|---|---|---|---|---|
| Binder D (45%) | 191.4 | 191.4 | 191.4 | 191.4 | 191.4 |
| Solvent | 8.6 | 8.6 | 8.6 | 8.6 | 8.6 |
| Binder B | | 20.0 | 40.0 | | |
| Binder C | | | | 13.3 | 26.6 |
| Water | 15.4 | 15.4 | 15.4 | 15.4 | 15.4 |
| **Total** | **215.4** | **235.4** | **255.4** | **228.7** | **242.0** |
| | | | | | |
| Polymer "soft-type A" dry | 86.3 | 86.3 | 86.3 | 86.3 | 86.3 |
| Hardener "hard-type 1" dry | | 6.0 | 12.0 | | |
| Hardener "hard-type 2" dry | 0.0 | | | 6.0 | 12.0 |
| **Total dry** | **86.3** | **92.3** | **98.3** | **92.3** | **98.3** |
| | | | | | |
| Polymer "soft-type A" dry % | 100.0 | 93.5 | 87.8 | 93.5 | 87.8 |
| Hardener "hard-type 1" dry % | | 6.5 | 12.2 | | |
| Hardener "hard-type 2" dry % | | | | 6.5 | 12.2 |
| **Total dry %** | **100.0** | **100.0** | **100.0** | **100.0** | **100.0** |

Notes
1. Binder D was a 45% solids aqueous binder containing a resin-supported 50:50, BA/Sty polymer having a T_{g} of 45°C, a film forming temperature of 19°C, an acid number of 105 and M_{w} 250,000.
2. The solvent was TEXANOL (2,2,4-trimethyl-1,3-pentanediol, mono-isobutyrate ester; available from Eastman Co., Kingsport TN)
3. The thickener was QR2020 (available from Rohm and Haas Co., Philadelphia, PA), a polyurethane thickener.

### FILM APPLICATION = 90 µ WET

| HARDNESS König 6° - 3° | | | | | |
|---|---|---|---|---|---|
| Drying time = 1 H | 41 | 54 | 65 | 41 | 35 |
| Drying time = 24 H | 55 | 77 | 88 | 60 | 42 |
| Drying time = 48 H | 55 | 85 | 89 | 60 | 52 |
| | | | | | |

| Water resistance (time min.) | | | | | |
|---|---|---|---|---|---|
| Drying time = 1 H | 1.5 | 0.5 | 0.5 | 0.5 | 0.5 |
| Drying time = 24 H | 3 | 3 | 5 | 5 | 1.5 |
| Drying time = 48 H | 4 | 4 | 6 | 6 | 2 |

### FILM APPLICATION = 30 µ WET

| HARDNESS Konig 6°- 3° | | | | | |
|---|---|---|---|---|---|
| Drying time = 1H | 87 | 110 | 115 | 106 | 70 |
| Drying time = 24H | * | | | | |
| Drying time = 48 H | * | | | | |

| | | | | | |
|---|---|---|---|---|---|
| * not measured | | | | | |

| Water resistance (time min.) | | | | | |
|---|---|---|---|---|---|
| Drying time = 1 H | 1.5 | 1.5 | 0.5 | 0.5 | 0.5 |
| Drying time = 24 H | 3 | 3 | 5 | 5 | 1.5 |
| Drying time = 48 H | 4 | 4 | 6 | 6 | 2 |

### Gloss Measurement

To the previous formulation 1.5% of a red colorant slurry from CPS Color is added so that gloss measurement can be evaluated.

| **Angle** | **20°** | | **60°** | | **85°** | |
|---|---|---|---|---|---|---|
| B1 | **86** | 84.8 | **92.4** | 91.9 | **98.8** | 98.2 |
| B2 | **84.6** | 84.1 | **91.9** | 92.2 | **98.6** | 98.6 |
| B3 | **87.1** | 87.4 | **96.6** | 95.8 | **99.7** | 99.5 |
| B4 | **88.9** | 89.1 | **93.9** | 95.5 | **98.9** | 99 |
| B5 | **87.9** | 90.7 | **95.4** | 93.8 | **97.2** | 97.1 |

### Example 3

| Ingredients / sample ref. | A1 | A2 | A3 | A4 | A5 |
|---|---|---|---|---|---|
| Binder E (50 %) | 185.8 | 185.8 | 185.8 | 185.8 | 185.8 |
| Solvent | 13.7 | 13.7 | 13.7 | 13.7 | 13.7 |
| Defoamer | 0.2 | 0.2 | 0.2 | 0.2 | 0.2 |
| Ammonia (28%) | 0.4 | 0.4 | 0.4 | 0.4 | 0.4 |
| Binder B (30%) | | 17.7 | 35.4 | | |
| Binder C (45%) | | | | 11.8 | 23.6 |
| Thickener | | 1.0 | 1.0 | 1.0 | 1.0 |
| **Total** | **200.0** | **218.7** | **236.4** | **212.8** | **224.61** |
| | | | | | |
| Polymer "soft-type A" dry | 94.8 | 94.81 | 94.8 | 94.8 | 94.8 |
| Hardener "hard-type 1" dry | | 5.3 | 10.6 | | |
| Hardener "hard-type 2" dry | 0.0 | | | 5.3 | 10.6 |
| **Total dry** | **94.8** | **100.1** | **105.4** | **100.1** | **105.4** |
| | | | | | |
| Polymer "soft-type A" dry % | 100.0 | 94.7 | 89.9 | 94.7 | 89.9 |
| Hardener "hard-type 1" dry % | | 5.3 | 10.1 | | |
| Hardener "hard-type 2" dry % | | | | 5.3 | 10.1 |
| **Total dry %** | **100.0** | **100.0** | **100.0** | **100.0** | **100.0** |

Notes
1. Binder E was a 50% solids aqueous binder containing a 67.2 MMA/31.4 BA/1.2 MAA polymer having a Tg of 53°C, a film forming temperature of 28°C, and M_{w} 400,000.
2. The solvent was TEXANOL (2,2,4-trimethyl-1,3-pentanediol, mono-isobutyrate ester; available from Eastman Co., Kingsport TN)
3. The thickener was QR2020 (available from Rohm and Haas Co., Philadelphia, PA), a polyurethane thickener.
4. The defoamer was NOPCO NDW (available from Cognis Co. in France), a sulfated castor oil.

### FILM APPLICATION = 90 µ WET

| HARDNESS König 6°- 3° | | | | | |
|---|---|---|---|---|---|
| Drying time = 1 H | 32 | 30 | 38 | 34 | 35 |
| Drying time = 24 H | 36 | 36 | 36 | 44 | 43 |
| Drying time = 48 H | 40 | 41 | 50 | 50 | 48 |
| | | | | | |

| Water resistance (time min.) | | | | | |
|---|---|---|---|---|---|
| Drying time = 1 H | 3.5 | 0.5 | 0.5 | 0.5 | 0.5 |
| Drying time = 24 H | 10 | 10 | 10 | 10 | 2.5 |
| Drying time = 48 H | * | | | | |

| | | | | | |
|---|---|---|---|---|---|
| * not measured | | | | | |

### FILM APPLICATION = 30 µ WET

| HARDNESS König 6°- 3° | | | | | |
|---|---|---|---|---|---|
| Dryingtime = 1 H | 61 | 85 | 95 | 55 | 59 |
| Drying time = 24 H | * | | | | |
| Drying time = 48 H | * | | | | |

| | | | | | |
|---|---|---|---|---|---|
| * not measured | | | | | |

| Water resistance (time min.) | | | | | |
|---|---|---|---|---|---|
| Drying time = 1H | 1 | 1 | 1 | 0.5 | 0.5 |
| Drying time = 24 H | 3.5 | 3.5 | 3 | 2.5 | 2 |
| Drying time = 48 H | * | | | | |

| | | | | | |
|---|---|---|---|---|---|
| * not measured | | | | | |

### Gloss Measurement

To the previous formulation 1.5% of a red colorant slurry from CPS Color is added so that gloss measurement can be evaluated.

| **Angle** | **20°** | | **60°** | | **85°** | |
|---|---|---|---|---|---|---|
| C1 | **82.3** | 82.1 | **87.6** | 87.4 | **98.4** | 98 |
| C2 | **83.8** | 83.5 | **88.3** | 87.5 | **98.1** | 98.1 |
| C3 | **80.1** | 80.1 | **87.2** | 87 | **97.5** | 98 |
| C4 | **82.8** | 83.3 | **87.1** | 87.9 | **97.5** | 98 |
| C5 | **80.3** | 78.3 | **87** | 86.5 | **97.5** | 97.2 |

### Example 4

The ratio between Binder B and Binder A was varied, and the water resistance and the film hardness were assessed.

| Ingredients / sample ref. | D1 | D2 | D3 | D4 |
|---|---|---|---|---|
| Binder A (40%) | 186.8 | 186.8 | 186.8 | 186.8 |
| Dowanol DPnB | 12.6 | 12.6 | 12.6 | 12.6 |
| Binder B (30%) | 44.0 | 84 | 165 | 266 |
| Thickener QR2020 | 3.6 | 8.9 | 17.8 | 22.6 |
| **Total** | **247.0** | **292.3** | **382.2** | **488.0** |
| | | | | |
| Binder A Dry Film | 85.0 | 74.8 | 60.2 | 48.4 |
| Binder B dry film | 15.0 | 25.2 | 39.8 | 51.6 |
| **Total dry %** | **100.0** | **100.0** | **100.0** | **100.0** |

### FILM APPLICATION = 90 µ WET

**HARDNESS König 6°- 3°**

| | | | | |
|---|---|---|---|---|
| Drying time = 1 H | 36 | 60 | 60 | 90 |
| Drying time = 24H | 95 | 96 | 110 | 120 |
| Drying time = 48 H | * | | | |

| | | | | |
|---|---|---|---|---|
| * not measured | | | | |

**Water resistance (time min.)**

| | | | | |
|---|---|---|---|---|
| Drying time = 1 H | 5 | 2 | 1 | 1 |
| Drying time = 24 H | 15 | 8 | 7 | 4 |
| Drying time = 48 H | * | | | |

| | | | | |
|---|---|---|---|---|
| * not measured | | | | |

Clearly, as the level of Binder B ("hard-type" polymer) increases, the film hardness increases, and the water resistance decreases, which indicates that it will be easy to remove the nail varnish.

### Example 5

To the previous samples was added 0.4% of dye in order to visualize the film removability. The formulations were then applied on the nail of one panelist. After two hours removability was assessed using different removers, and rated as "poor," moderate ("mod") or "good."

| | D1 | D2 | D3 | D4 |
|---|---|---|---|---|
| TAP WATER 35°C | poor | poor | mod | **good** |
| TAP WATER 35 °C+ Commercial Liquid hand soap | poor | mod | **good** | **good** |
| TAP WATER 35°C + Commercial Hand Dish wash liquid + pH=9 | mod | **good** | **good** | **good** |
| commercial solvent removal | **good** | **good** | **good** | **good** |

## Claims

1. A method of coating human nails with a polymer film; said method comprising steps of:
(a) applying to the human nails a composition comprising: (i) a first polymer having M_{w} greater than 20,000 and a T_{g} less than 70°C; (ii) a second polymer having M_{w} less than 15,000 and a Tg greater than 90°C; and (iii) water; wherein the composition contains less than 10% of organic solvents; and
(b) allowing the composition to dry until the polymer film has formed.

2. The method of claim 1 in which the composition comprises 40-96% of the first polymer and 4-60% of the second polymer, based on dry weight of polymeric material in the composition.

3. The method of claim 2 in which the composition contains less than 8% of organic solvents, less than 4% of VOC solvents, and less than 10% inorganic material.

4. The method of claim 3 in which the first polymer has a M_{w} greater than 25,000 and a T_{g} less than 60°C, and the second polymer has M_{w} less than 10,000 and a Tg greater than 100°C.

5. The method of claim 4 in which the first polymer and the second polymer are acrylic-styrene copolymers.

6. The method of claim 5 in which the composition comprises 40-90% of the first polymer and 10-60% of the second polymer, based on dry weight of polymeric material in the composition.

7. A composition suitable for use as a nail polish comprising:
(a) a first polymer having M_{w} greater than 20,000 and a Tg less than 70°C;
(b) a second polymer having M_{w} less than 15,000 and a Tg greater than 90°C; and
(c) water;
wherein the composition contains less than 10% of organic solvents and less than 10% inorganic material; and wherein the composition comprises 40-96% of the first polymer and 4-60% of the second polymer, based on dry weight of polymeric material in the composition.

8. The composition of claim 7 in which the composition contains less than 8% of organic solvents and less than 4% of VOC solvents.

9. The composition of claim 8 in which the first polymer has M_{w} greater than 25,000 and a Tg less than 60°C, and the second polymer has M_{w} less than 10,000 and a Tg greater than 100°C; and the composition comprises 40-90% of the first polymer and 10-60% of the second polymer, based on dry weight of polymeric material in the composition.

10. The composition of claim 9 in which the first polymer and the second polymer are acrylic-styrene copolymers.
